# EUROPEAN PATENT APPLICATION

(11) **EP 4 592 398 A1**
(43) Date of publication of application: **30.07.2025**
(21) Application number: 24837520.6
(22) Date of filing: 20.09.2024
(51) Int. Cl.: C12Q 1/6806, C12Q 1/70, C12Q 1/686, C12R 1/93

(54) **NUCLEIC ACID DETECTION METHOD**

(30) Priority: 07.12.2023 CN 202311674732; 07.12.2023 CN 202311677300
(71) Applicant: Sansure Biotech Inc., Changsha, Hunan 410205 (CN)
(72) Inventor: DAI, Lizhong, Changsha Hunan 410205 (CN); SUN, Qingzhi, Changsha Hunan 410205 (CN); TAN, Deyong, Changsha Hunan 410205 (CN); XU, Siyao, Changsha Hunan 410205 (CN); WANG, Jian, Changsha Hunan 410205 (CN); GUO, Jun, Changsha Hunan 410205 (CN); JI, Bozhi, Changsha Hunan 410205 (CN); WU, Kang, Changsha Hunan 410205 (CN); LIU, Jia, Changsha Hunan 410205 (CN)
(74) Representative: RGTH
(86) International application number: PCT/CN2024/120027
(87) International publication number: WO 2025/118777

(57) **Abstract**

The application belongs to the field of molecular biological detection, particularly relates to a nucleic acid detection method, and more particularly relates to a method for rapidly extracting and detecting nucleic acids. The application provides a nucleic acid detection method which includes the following steps: S1, mixing a to-be-tested sample with a direct amplification preservation solution, and performing ultrasonic treatment to obtain a sample nucleic acid solution; S2, adding the sample nucleic acid solution obtained in step S1 into an amplification reaction solution to obtain a mixed solution; S3, carrying out amplification reaction by the mixed solution, and detecting nucleic acids; where the direct amplification preservation solution includes a protective agent, a nuclease inhibitor, a surfactant, a buffer system and a bacteriostatic agent. By the method provided by the application, the whole detection process is simple and less affected, and the detection time is short.

## Description

### CROSS REFERENCING OF THE RELATED APPLICATIONS

This application is based on Chinese patent application CN202311674732.4, filed on December 7, 2023, titled "Nucleic Acid Detection Method", and Chinese patent application CN202311677300.9, filed on December 7, 2023, titled "Sample Preservation Composition", and claims their priorities. The contents of these Chinese patent applications are incorporated herein by reference in their entirety.

### FIELD OF THE INVENTION

The present application belongs to the field of molecular biology detection, and in particular, relates to a nucleic acid detection method, and a sample preservation composition used in this method; and more particularly, to a method for rapidly extracting and detecting nucleic acids, and a sample preservation composition used in this method for direct amplification without extraction.

### BACKGROUND OF THE INVENTION

Detecting pathogens or disease markers through molecular diagnostics is one of the important auxiliary methods for clinical diagnosis. Currently, molecular diagnosis usually comprises three parts: sample collection, storage and transportation; sample extraction; and nucleic acid detection. However, molecular diagnosis has certain requirements on the purity and integrity of a nucleic acid sample. When the quality of nucleic acid cannot meet the requirements, it will affect the accuracy and detection limit of subsequent detection results. The sample collection and transportation process, extraction method and efficiency will all have an impact on nucleic acids. In addition, generally nucleic acid extraction has relatively high requirements on instruments and personnel, and the entire extraction process takes a long time. From collecting samples to detecting the results, the entire process is cumbersome and time-consuming, taking more than 4 hours. Existing nucleic acid detection has problems such as complex detection process, easy contamination during operation, and long detection time, which does not meet the increasing demand for accurate and rapid detection.

The commonly used sample preservation method currently is to use Hank's solution or phosphate buffer solution for preservation, and the disadvantages are as follows: 1. it requires a subsequent nucleic acid extraction step, which is time-consuming and prone to contamination; 2. the preservation effect of various preservation solutions is of poor quality, and RNA is easily degraded; 3. the samples need to be stored at low temperatures after sampling, and the storage time is short.

After sampling, conventional extraction-free sample preservation solution contains more lysis components, which have a stronger lysis effect and cause greater damage to the structure of nucleic acids. After a short storage period of the sample, part of the nucleic acids will degrade, thereby reducing the detection effect and leading to the risk of false negatives. On the other hand, after the conventional extraction-free sample preservation solution is used for crude extraction of the sample, the content of inhibitor is high because there is no purification step, which has a greater impact on subsequent nucleic acid detection.

Therefore, there is an urgent need to develop a method that can quickly inactivate infectious viruses; thus, the subsequent samples do not require nucleic acid extraction, which reduces the steps in the entire process without affecting subsequent nucleic acid detection, especially RNA detection, and can be stored at room temperature for a long time. There is also a need for a cell preservation solution that can be directly used for detection, a nucleic acid detection method that can shorten the overall detection time.

### BRIEF DESCRIPTION OF THE INVENTION

In view of this, in a first aspect, the present application provides a nucleic acid detection method, including the following steps:
S1, mixing a to-be-tested sample with a direct amplification preservation solution, and performing ultrasonic treatment to obtain a sample nucleic acid solution;
S2, adding the sample nucleic acid solution obtained in step S1 into an amplification reaction solution to obtain a mixed solution;
S3, carrying out amplification reaction by the mixed solution, and detecting nucleic acids;
where the direct amplification preservation solution includes: a protective agent, a nuclease inhibitor, a surfactant, a buffer system, and a bacteriostatic agent.

The "direct amplification" as used herein refers to the release and amplification of nucleic acid without extraction of samples. It refers to direct amplification and detection of sample nucleic acids without the need for nucleic acid extraction or purification of the sample.

The direct amplification preservation solution used in the present application has the functions of sample preservation, sample inactivation and nucleic acid release. By combining ultrasonic treatment, the sample extraction process can be accelerated and thoroughly mixed in real-time, thereby shortening the sample nucleic acid release and extraction time to within 3 min. The sample nucleic acid solution after ultrasonic treatment is directly added to the amplification reaction solution for amplification detection, which greatly shortens the detection time. The entire process can be completed within 1 hour, thereby significantly improving the detection efficiency and meeting the needs of rapid nucleic acid detection.

In some particular embodiments, in step S1, the to-be-tested sample is mixed with the direct amplification preservation solution in a container with a pipetting function; and the sample nucleic acid solution is directly added to the amplification reaction premix through the container. In this way, the traditional method of adding samples using pipettes of different specifications and matching Tip heads of different specifications for pipetting can be avoided, which simplifies the operation. At the same time, it can avoid the contamination caused by the process of aspirating, pipetting, and blowing the liquid, thereby saving costs.

Further, the direct amplification preservation solution is loaded into a container with a pipetting function, and a to-be-tested sample is added to the direct amplification preservation solution for mixing and ultrasonic treatment. For example, the container can be a pipette, a centrifuge tube, etc.

In some particular embodiments, the pipette is a vesicle pipette. Further, the vesicle pipette has a quantitative pipetting function.

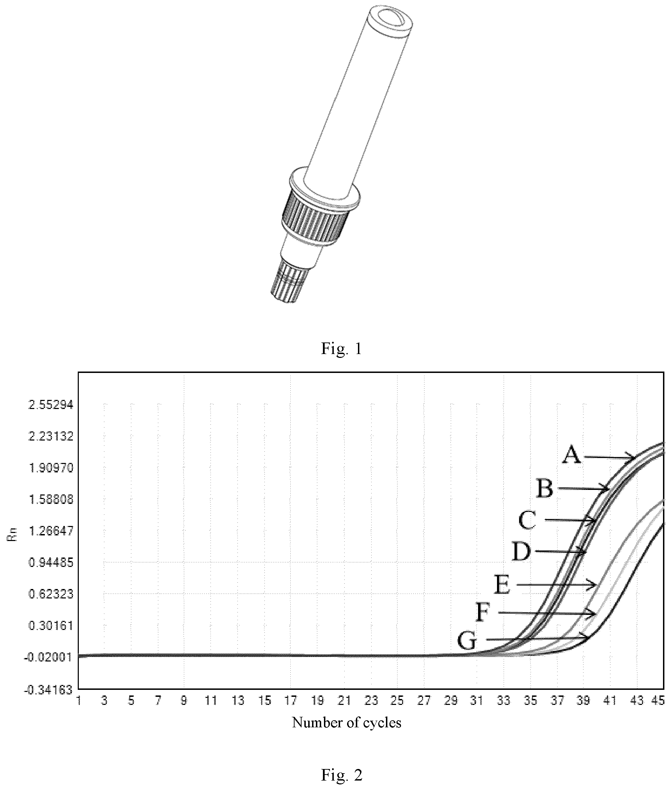

In a particular embodiment, as shown in Fig. 1, the vesicle pipette includes: a tube body, a dripper, a cover body and an extrusion body, where the dripper is detachably arranged at a first end of the tube body, the cover body is detachably mounted on the outer peripheral side of the dripper for sealing the liquid outlet on the dripper, the extrusion body is arranged at a second end of the tube body to form a flexible vesicle portion, and an extrusion cavity for communicating with the cavity of the tube body is formed inside the flexible vesicle portion. Each time the flexible vesicle portion is pressed to deform it into a planar state, so that the space inside vesicle pipette can only be reduced by a preset size each time. Accordingly, the liquid inside the vesicle pipette can only be squeezed outward to a preset volume. The pipette has a simple structure and is not prone to pipetting deviation due to fatigue operation when used for pipetting, thereby better ensuring the accuracy of quantitative pipetting, and improving the precision of pipetting.

Thus, the vesicle pipette can be used to load direct amplification preservation solution, add a to-be-tested sample for ultrasonic treatment, and achieve quantitative pipetting, while avoiding sample contamination caused by the use of additional pipettes during sample transfer.

In some particular embodiments, the protective agent includes: sucrose, choline chloride, mannitol, polyvinyl pyrrolidone, and glycerol.

In some particular embodiments, in the direct amplification preservation solution, the concentration of sucrose is 0.15-1.5% (w/v), the concentration of choline chloride is 1-10 mM, the concentration of polyvinyl pyrrolidone is 1-10 mM, the concentration of mannitol is 0.045-0.45% (v/v), and the concentration of glycerol is 0.05-0.5% (v/v).

In the definition of the present application, the term "w/v" means mass to volume concentration, and the term "v/v" means volume fraction, where w is measured in g, and v is measured in mL. In some particular embodiments, the concentration of the nuclease inhibitor is 0.01-0.1% (v/v), for example, 0.01%, 0.02%, 0.03%, 0.04%, 0.05%, 0.06%, 0.07%, 0.08%, 0.09%, or 0.1%.

The nuclease inhibitor is at least one of: RNasin, diethyl pyrophosphate, or guanidine isothiocyanate. In a particular embodiment, the nucleic acid inhibitor is RNasin.

In some particular embodiments, the concentration of the surfactant is 0.01-0.5% (v/v), for example, 0.01%, 0.02%, 0.05%, 0.1%, 0.2%, 0.3%, 0.4%, or 0.5%. The surfactant is at least one of: Triton X-100, Tween 20, sodium dodecyl alcohol ether sulfate or sodium dodecyl sulfate. In a particular embodiment, the surfactant is Triton X-100 and/or Tween 20.

In some particular embodiments, the concentration of the bacteriostatic agent is 0.01-0.1% (v/v), and the concentration of the buffer system is 80-120 mM. The bacteriostatic agent is Proclin 300 and/or potassium sorbate. In a particular embodiment, the bacteriostatic agent is Proclin 300.

In some particular embodiments, the buffer system is at least one of: Tris-HCl buffer, phosphate buffer, or TE buffer. In a particular embodiment, the buffer system is Tris-HCl buffer. Further, in the direct amplification preservation solution, the concentration of the buffer system is 80-120 mM.

In some particular embodiments, a pH value of the direct amplification preservation solution is 11-13; preferably, the pH value of the direct amplification preservation solution is about 12.

The use of the direct amplification preservation solution can achieve better detection results after the direct amplification method of the present application.

In some particular embodiments, the frequency of ultrasound in step S1 is 30-80 kHz, and the duration is 30-150S; preferably, the frequency of the ultrasound is 40 kHz, and the duration is 120S.

In some particular embodiments, the ultrasonic treatment includes applying ultrasound to the side or bottom of the container; preferably, the ultrasound is applied to the sides of the container.

In some particular embodiments, in step S2, the amplification reaction solution is a PCR reaction solution, where the PCR reaction solution includes any one or more of: primers, probes, Mg²⁺, dNTPs, Taq enzyme, MMLV enzyme (reverse transcriptase), or PCR buffer.

Further, the PCR reaction solution includes Mg²⁺ 4mM-8mM, dNTPs 0.2-0.6mM, Taq enzyme 5-30U, primers 200-600nM, probes 100-500nM, and RNasin 1-20U. In some embodiments, the PCR reaction solution further includes reverse transcriptase 5-30U.

In some particular embodiments, the PCR reaction solution is a fully premixed reaction solution with a pH value ranging from 8 to 9.

Preferably, the PCR reaction solution includes Mg²⁺ 4mM, dNTPs 0.4mM, MMLV 5U, Taq enzyme 15U, primers 200nM, probes 100nM, and RNasin 5U. The use of this PCR reaction solution can achieve the best preservation effect.

In some particular embodiments, in step S3, the detection is PCR, and the duration of the PCR does not exceed 60 min. Further, the duration of the PCR is 30-60 min.

Further, the PCR procedure is as follows:

| Procedure | | |
|---|---|---|
| Temperature | Time | Number of cycles |
| 50°C | 3min | 1 |
| 95°C | 30s | 1 |
| 95°C | 10s | 45 |
| 60°C | 20s | |

The method according to the present application can quickly and fully process samples, release nucleic acids, easily and accurately transfer sample nucleic acids, and efficiently and quickly implement amplification detection, thereby simplifying the entire detection process, reducing pollution, shortening detection time, and ensuring the accuracy of detection results.

In another aspect, the present application provides a sample preservation composition including a protective agent, where the protective agent includes: sucrose, choline chloride, mannitol, polyvinyl pyrrolidone, and glycerol.

The applicant creatively discovered that, the use of the sample preservation composition can enable the sample to be stored at room temperature for a long time, and its simple composition will not affect its performance, thereby well maintaining its detection sensitivity and stability.

Further, the concentrations of the sucrose, choline chloride, mannitol, polyvinyl pyrrolidone and glycerol in the protective agent are as follows: the concentration of sucrose is 15% (w/v), the concentration of choline chloride is 0.1M, the concentration of polyvinyl pyrrolidone is 0.1M, the concentration of mannitol is 4.5% (v/v), and the concentration of glycerol is 5% (v/v).

After the protective agent is formulated into a sample preservation composition, in the sample preservation composition, the concentration of sucrose is 0.15-1.5% (w/v), the concentration of choline chloride is 1-10 mM, the concentration of polyvinyl pyrrolidone is 1-10 mM, the concentration of mannitol is 0.045-0.45% (v/v), and the concentration of glycerol is 0.05-0.5% (v/v).

In the definition of the present application, the term "w/v" means mass to volume concentration, and the term "v/v" means volume fraction, where w is measured in g, and v is measured in mL.

Further, the sample preservation composition further includes any one or more of: a nuclease inhibitor, a surfactant, a buffer system, or a bacteriostatic agent.

In a particular embodiment, the sample preservation composition includes a protective agent, a nuclease inhibitor, a surfactant, a buffer system, and a bacteriostatic agent.

In some particular embodiments, the concentration of the protective agent is 1-10% (v/v), for example, 1%, 2%, 3%, 4%, 5%, 6%, 7%, 8%, 9%, or 10%. Preferably, the concentration of the protective agent is 1-5% (v/v); and more preferably, the concentration of the protective agent is 1%.

In some particular embodiments, the concentration of the nuclease inhibitor is 0.01-0.1% (v/v), for example, 0.01%, 0.02%, 0.03%, 0.04%, 0.05%, 0.06%, 0.07%, 0.08%, 0.09%, or 0.1%. The nuclease inhibitor can be any one or more of: RNasin, diethyl pyrophosphate, guanidine isothiocyanate, or the like. In a particular embodiment, the nucleic acid inhibitor is RNasin.

In some particular embodiments, the concentration of the surfactant is 0.01-0.5% (v/v), for example, 0.01%, 0.02%, 0.05%, 0.1%, 0.2%, 0.3%, 0.4%, or 0.5%. The surfactant can be any one or more of: Triton X-100, Tween 20, sodium dodecyl alcohol ether sulfate, or sodium dodecyl sulfate. In a particular embodiment, the surfactant is Triton X-100 and/or Tween 20.

In some particular embodiments, the concentration of the bacteriostatic agent is 0.01-0.1% (v/v), for example, 0.01%, 0.02%, 0.05%, 0.07%, 0.08%, 0.09%, or 0.1%. The bacteriostatic agent can be any one or more of: Proclin 300, potassium sorbate, or the like. In a particular embodiment, the bacteriostatic agent is Proclin 300.

In some particular embodiments, the buffer system can be any one or more of: Tris-HCl buffer, phosphate buffer, TE, or the like. In a particular embodiment, the buffer system is a Tris-HCl buffer with a pH of 8.0-8.5. Further, the concentration of the buffer system is 80-120 mM.

The sample preservation composition of the present application can be used for detection without the need for lysis and nucleic acid extraction steps, and can enable samples to be stored at room temperature for a long time, and its simple composition will not affect the performance, thereby well maintaining the detection sensitivity and stability.

In still another aspect, the present application provides a PCR reaction composition, which includes the sample preservation composition as described above.

Further, the PCR reaction composition further includes a sample and PCR reaction reagents.

Further, the sample is a biological sample containing nucleic acids; preferably, a biological sample containing RNA, for example, viruses whose genetic material is RNA.

Further, the PCR reaction reagents further include a primer and probe for PCR.

Further, the PCR reaction reagents further include any one or more of: dNTPs, reverse transcriptase, DNA polymerase, or PCR buffer.

In the definition of the present application, the term "PCR reaction composition" refers to a mixture capable of detecting nucleic acids by PCR.

In a particular embodiment, the PCR reaction composition includes the sample preservation composition as described above, a primer and probe, dNTPs, reverse transcriptase, DNA polymerase, and PCR buffer.

In still another aspect, the present application provides an extraction-free nucleic acid detection kit, which includes the sample preservation composition as described above. Further, the nucleic acid detection kit is a PCR detection kit, which further includes the PCR reaction composition as described above.

In still another aspect, the present application provides use of the above-mentioned composition in the preparation of an extraction-free nucleic acid detection kit. Further, the nucleic acid detection kit is a PCR detection kit.

In still another aspect, the present application provides a method for extraction-free PCR detection, which includes a step of mixing a sample with the above-mentioned sample preservation composition and the above-mentioned PCR reaction reagent.

The PCR reaction reagents include, for example, dNTPs, reverse transcriptase, DNA polymerase, and PCR buffer; further, it can also include primers and probes.

### BRIEF DESCRIPTION OF THE DRAWINGS

Fig. 1 is a schematic diagram of a vesicle pipette.
Fig. 2 is the amplification curves of samples treated with different compositions and methods (from A to G are magnetic bead method, preservation solutions for direct amplification 1-6, respectively).
Fig. 3 is a diagram showing the detection results of sample 1 treated with different treatment methods.
Fig. 4 is a diagram showing the detection results of sample 2 treated with different treatment methods.
Fig. 5 is the detection results of detecting influenza A virus from samples processed by the method of the present application and the magnetic bead method.
Fig. 6 is the detection results of detecting influenza B virus from samples processed by the method of the present application and the magnetic bead method.
Fig. 7 is the detection results of detecting novel coronavirus virus from samples processed by the method of the present application and the magnetic bead method.
Fig. 8 is the detection results of detecting Mycoplasma pneumoniae from samples processed by the method of the present application and the magnetic bead method.

### DETAILED DESCRIPTION OF THE INVENTION

The present application will be described in detail below in conjunction with particular embodiments and examples, and the advantages and various effects of the present application will be more clearly presented. Those skilled in the art should understand that, these particular embodiments and examples are used to illustrate the present application rather than to limit the present application.

At present, the main methods used for extracting nucleic acids from respiratory pathogens are: centrifugal column nucleic acid extraction method, magnetic bead method, and alkaline lysis method. Particularly, the centrifugal column nucleic acid extraction method takes up to 1-2h because it requires specific adsorption and repeated washing of impurities. The magnetic bead method for extracting nucleic acids has become the mainstream method in the industry due to its high degree of automation, large sample throughput, good nucleic acid concentration and purity, and high safety; however, the extraction process is easily contaminated and takes 30 min to 2 hours. The alkaline lysis method is a relatively simplified extraction method, which uses a strong base to lyse a sample; nucleic acids after sample lysis in this method are easily degraded, have a very short storage time, cannot be retested, and the method takes about 10 min. Traditional PCR reaction reagents usually include a primer and probe, DNA polymerase, RNA polymerase, PCR buffer and other components packaged separately; they need to be prepared before use, and the operation is complicated. At the same time, the traditional PCR amplification program is as follows: 50°C, 30min; 95°C, 5min (95°C, 1min; 60°C, 30sec; 72°C, 30sec) for 45 cycles, and the duration of the PCR detection takes about 2h.

In order to meet the demand for rapid clinical detection, the inventors of the present application have developed a novel nucleic acid detection method. Through the detection scheme of direct amplification and ultrasonic treatment + pipette + premixing + rapid amplification, the detection time can be greatly shortened, the detection process can be shortened to within 1 hour, the contamination caused by sample nucleic acid transfer can be reduced, and the detection efficiency can be improved, thereby ensuring the accuracy and reliability of the detection results.

### Example 1

### Preparation of Direct amplification preservation solution

### Step 1: preparation of protective agent.

Protective agent No.1: 15 g of sucrose, 1.3963 g of choline chloride, and 1.1114 g of polyvinyl pyrrolidone were added in sequence into 50 mL of sterile water; after dissolution, 4.5 mL of mannitol and 5 mL of glycerol were added, then making up to 100 mL with sterile water.

Protective agent No.2: 15 g of sucrose, 1.3963 g of choline chloride, and 3.423 g of trehalose were added in sequence into 50 mL of sterile water; after dissolution, 4.5 mL of mannitol and 5 mL of glycerol were added, then making up to 100 mL with sterile water.

Protective agent No.3: 15 g of sucrose and 1.1114 g of polyvinyl pyrrolidone were added in sequence into 50 mL of sterile water; after dissolution, 4.5 mL of mannitol, 5 mL of glycerol, and 1 mL of dimethyl sulfoxide were added, then making up to 100 mL with sterile water.

Protective agent No.4: 15 g of sucrose was added into 50 mL of sterile water; after dissolution, 4.5 mL of mannitol and 5 mL of glycerol were added, then making up to 100 mL with sterile water.

### Step 2: preparation of direct amplification preservation solution.

Protective agent No.1 was used to prepare direct amplification preservation solution No.1: 1 mL of protective agent No.1, 5 µL of Triton X-100, 5 µL of Tween 20, 10 µL of RNasin, 10 µL of Proclin300, and 1.2608 g of Tris-HCl solid were added into 50 mL of sterile water, then adjusting pH, and making up to 100 mL with sterile water to prepare direct amplification preservation solution No.1.

Protective agent No.1 was used to prepare direct amplification preservation solution No.2: 5 mL of protective agent No.1, 50 µL of Triton X-100, 50 µL of Tween 20, 50 µL of RNasin, 50 µL of Proclin 300, and 1.576 g of Tris-HCl solid were added into 50 mL of sterile water, then adjusting pH, and making up to 100 mL with sterile water to prepare direct amplification preservation solution No.2.

Protective agent No.1 was used to prepare direct amplification preservation solution No.3: 10 mL of protective agent No.1, 250 µL of Triton X-100, 250 µL of Tween 20, 100 µL of RNasin, 100 µL of Proclin300, and 1.8912g of Tris-HCl solid were added into 50mL sterile water, then adjusting pH, and making up to 100mL with sterile water to prepare direct amplification preservation solution No.3.

Protective agents Nos.2-4 were used to prepare preservation solutions for direct amplification Nos.4-6: 1 mL of protective agents Nos.2-4 were added to 3 portions of 50 mL of sterile water respectively, and then continuing to add 5 µL of Triton X-100, 5 µL of Tween 20, 10 µL of RNasin, 10 µL of Proclin 300, and 1.2608 g of Tris-HCl solid to each portion, then adjusting the pH and making up to 100 mL with sterile water to prepare preservation solutions for direct amplification Nos.4-6.

The influenza A virus samples of 1*10⁵ copies/mL were chemically treated using the 6 preservation solutions for direct amplification Nos.1-6 prepared above, diluted to 1*10⁴ copies/mL, and then subjected to ultrasonic treatment (40 kHz, 120 s).

In addition, nucleic acid extraction and purification reagent S10015 produced by Sansure Biotech Co., Ltd. was used to extract nucleic acid from influenza A virus samples using magnetic beads.

The detection was performed using the universal nucleic acid detection kit for influenza A virus produced by Sansure Biotech Co., Ltd. The reaction system is as follows: 20 µL of treated sample + 30 µL of PCR reaction solution, and the detection was performed on the SLAN-96P fully automatic medical PCR analysis system. The PCR procedure is shown in Table 1 below.

**Table 1**

| Procedure | | |
|---|---|---|
| Temperature | Time | Number of cycles |
| 50°C | 3min | 1 |
| 95°C | 30s | 1 |
| 95°C | 10s | 45 |
| 60°C | 20s | |

The results are shown in Fig. 2, where A is the amplification result of the sample extracted by the magnetic bead method, and B-G are the amplification results of the samples treated with sample treatment solutions Nos. 1-6, respectively.

As shown in Fig. 2, the above six sample processing solutions can all be used for direct amplification without the need for additional nucleic acid extraction steps, and can all meet the detection requirements. But relatively speaking, the Ct value of the formula of sample processing solutions Nos. 1-3 is closer to the front, the fluorescence increment is higher, and the release effect of nucleic acid is better; and the result is not much different from that of the control group of magnetic bead extraction, which is basically consistent with the extraction effect of the magnetic bead method.

### Example 2

(1) Throat swabs were used to gently scrape epidermal mucosal cells from the surface of the human tongue or the buccal mucosa (cheeks on both inner sides of the oral cavity). Soak each of the swabs in a vesicle dropper (as shown in Fig. 1) containing 2 mL of the direct amplification preservation solution No.1 prepared in Example 1, break off the swab head, and cover tube caps tightly. The vesicle droppers were numbered as Nos. 1-13 respectively.
(2) The influenza A virus simulation sample stock solution was diluted to 1*10⁵ copies/mL (Sample 1), then added to the treatment solutions Nos. 1-13 prepared in the above step (1) to obtain samples Nos. 1-13.
(3) Samples Nos.1-13 in step (2) were placed in an ultrasonicator for ultrasonic treatment. The ultrasonic treatment conditions were shown in Table 2. After ultrasonic treatment, the samples were placed on ice for cooling for 1 min.

**Table 2**

| Number | No.1 | No.2 | No.3 | No.4 | No.5 | No.6 | No.7 |
|---|---|---|---|---|---|---|---|
| Sample processing | 40Khz, 120s | 40Khz, 80s | 40Khz, 150s | 30Khz, 150s | 60Khz, 80s | 80Khz, 30s | Without ultrasonic treatment |

| Number | No.8 | No.9 | No.10 | No.11 | No.12 | No. 13 | |
|---|---|---|---|---|---|---|---|
| Sample processing | 80Khz, 160s | 20Khz, 10s | 30Khz, 30s | 30Khz,6 0s | 80Khz, 100s | 80Khz, 1 50s | |

(4) Samples Nos.1-13 were detected using the universal nucleic acid detection kit for influenza A virus from Sansure Biotech Co., Ltd. After ultrasonic treatment, take a vesicle dropper to add a drop (20 µL) of sample into a PCR tube containing 30 µL of PCR premixed reaction solution, cover tube caps tightly and perform detection on the SLAN-96P fully automatic medical PCR analysis system. The detection procedure is shown in Table 2. The detection results are shown in Table 3.

In addition, referring to the above steps (1)-(4), the simulated samples of the novel coronavirus (Sample 2) and *Mycoplasma pneumoniae* (Sample 3) were processed respectively, and the novel coronavirus nucleic acid detection kit and *Mycoplasma pneumoniae* nucleic acid detection kit from Sansure Biotech Co., Ltd. were used for detection. The detection results are shown in Table 3.

**Table 3**

| Detection results (Ct) | Sample 1 | Sample 2 | Sample 3 |
|---|---|---|---|
| No.1 | 27.90 | 29.16 | 32.28 |
| No.2 | 30.01 | 33.07 | 34.25 |
| No.3 | 31.27 | 34.00 | 37.11 |
| No.4 | 30.29 | 33.48 | 34.00 |
| No.5 | 28.44 | 30.03 | 33.00 |
| No.6 | 30.49 | 34.30 | 35.75 |
| No.7 | 34.19 | 35.05 | 38.32 |
| No.8 | No Ct | No Ct | No Ct |
| No.9 | 38.27 | No Ct | No Ct |
| No.10 | 31.86 | 35.39 | 37.48 |
| No.11 | 32.67 | 34.54 | 35.90 |
| No.12 | 28.58 | 33.98 | 34.17 |
| No. 13 | 31.79 | 34.49 | 36.35 |

It can be seen from Table 3 that, when the sample is processed under the ultrasonic condition of the frequency of 30-80kHz for a duration of 30-150S, good detection effect can be obtained. Compared with sample No. 7 which has not been treated with ultrasound, their Ct values can be advanced by up to 6, and compared with the control samples of other ultrasound conditions, their detection effects are also better.

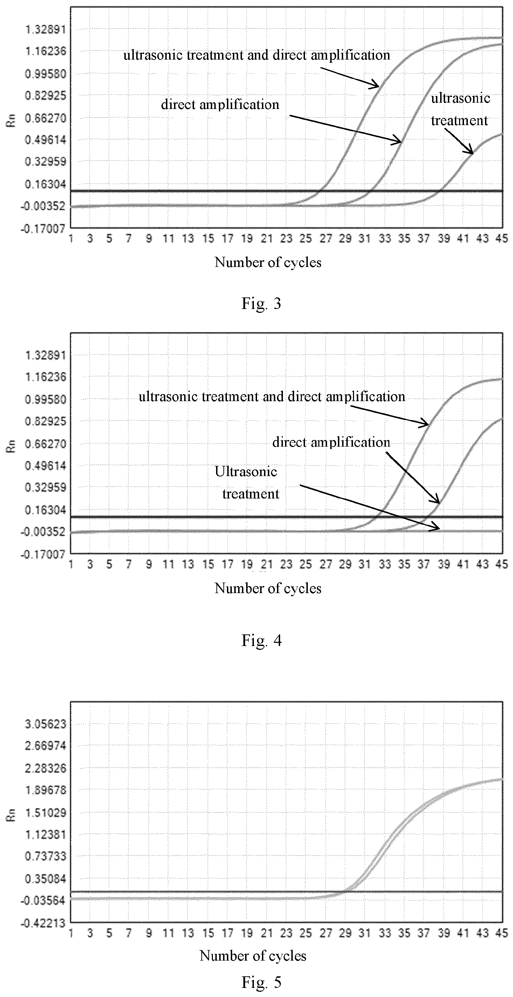

Further, as a control, the direct amplification preservation solution 1 was not used, and only ultrasonic treatment was performed to treat the Sample 1 and Sample 2. The results are shown in Table 4 and Figs. 3-4. It can be seen from Table 4 that, the detection effect of using the two methods to process a sample is better than that of using only ultrasonic treatment.

**Table 4**

| Detection results (Ct) | Sample 1 | Sample 2 |
|---|---|---|
| Ultrasonic treatment and direct amplification (a combination of physical + chemical treatment) | 26.39 | 32.16 |
| Only ultrasonic treatment (physical treatment) | 38.55 | No Ct |

### Example 3

Referring to the method of Example 2, the influenza A virus sample was chemically treated using the direct amplification preservation solution 1 of Example 1, followed by ultrasonic treatment (40 kHz, 120 s), and then the treated samples were mixed with the PCR reaction solutions (stored at -20°C for 6 months) shown in Table 5 below respectively, stored at -20°C according to the treatment time shown in Table 6, and finally performed detection on the SLAN-96P fully automatic medical PCR analysis system.

**Table 5**

| PCR reaction solution 1 | PCR reaction solution 2 | PCR reaction solution 3 | PCR reaction solution 4 |
|---|---|---|---|
| Mg²⁺ 4mM | Mg²⁺ 3mM | Mg²⁺ 4mM | Mg²⁺ 4mM |
| dNTPs 0.4mM | dNTPs 0.6mM | dNTPs 0.2mM | dNTPs 0.2mM |
| MMLV, 5U | MMLV, 10U | MMLV, 15U | MMLV, 15U |
| Taq enzyme, 15U | Taq enzyme, 10U | Taq enzyme, 20U | Taq enzyme, 30U |
| Primers, 200nM | Primers, 300 nM | Primers, 400 nM | Primers, 600 nM |
| Probs, 100 nM | Probs, 150 nM | Probs, 450 nM | Probs, 300 nM |
| Rnsin,5U | Rnsin,10U | Rnsin,15U | Rnsin,20U |
| PCR buffer (1x) | PCR buffer (1x) | PCR buffer (1x) | PCR buffer (1x) |

The results are shown in Table 6. It can be seen from Table 6 that, the preservation effect of PCR reaction solution 1 is the best.

**Table 6**

| Processing temperature | Storage at -20°C | | | |
|---|---|---|---|---|
| Processing time | 0 d | 2 months | 4 months | 6 months |
| Pre-mixed reaction solution 1 | 34.31 | 34.34 | 34.96 | 34.60 |
| Pre-mixed reaction solution 2 | 34.12 | 35.09 | 35.08 | 35.98 |
| Pre-mixed reaction solution 3 | 36.97 | 36.06 | 36.83 | 36.82 |
| Pre-mixed reaction solution 4 | 34.88 | 35.03 | 35.77 | 35.99 |

### Example 4: Detection of Different Pathogens by the Method of the Present Application and Magnetic Bead Method

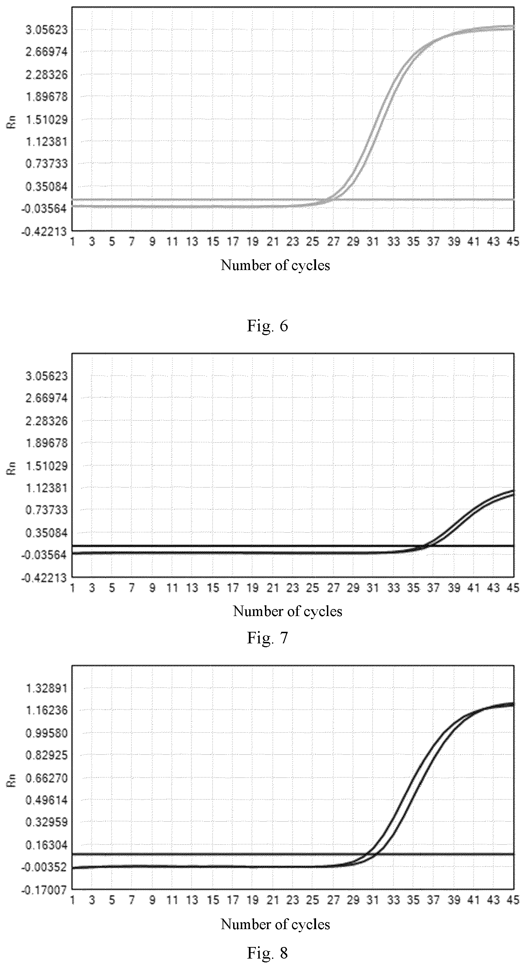

The nucleic acid detection method of the present application and the magnetic bead method were used to detect samples (influenza A virus, influenza B virus, novel coronavirus, *and Mycoplasma pneumoniae),* and the detection results are shown in Figs. 5-8. The results show that, the method of the present application is significantly effective, and is comparable to the effect of nucleic acid detection after nucleic acid extraction by the magnetic bead method. The detection reagents come from the novel coronavirus 2019-nCoV, influenza A virus, influenza B virus nucleic acid detection kit, Mycoplasma pneumoniae test kit, etc. from Sansure Biotech Co., Ltd.

Further, the detection times used by the two methods were compared, and the results are shown in Table 6 (taking influenza A virus samples as an example). The method described in the present application can shorten the detection process by at least 1-2 hours, making the entire detection faster.

**Table 6**

| Method | Nucleic acid extraction | Preparing PCR reaction solution, and mixing sample with PCR reaction solution | PCR | Total |
|---|---|---|---|---|
| Method of the application | 2 min | 2 min | No more than 60 min | No more than 70 min |
| Magnetic bead method | 30 min | 20 min | 120 min | 170 min |

### Example 5: Temperature Stability (Preservation) Effect of the Sample Preservation Composition of the Present Application

(1) Sample preservation solutions Nos.1, 2 and 3 were prepared using the protective agent No.1 in the above Example 1; while Control groups No. 4, 5, and 6 were prepared using the protective agents Nos.2-4 in the above Example 1
   Throat swabs were used to gently scrape the epidermal mucosal cells on the human tongue or buccal mucosa (cheeks on both inner sides of the mouth). Soak each of the swabs in 2 mL of Sample preservation solutions Nos.1-6, cover tube caps tightly, mix upside down for 90 seconds, and then perform instantaneous centrifugation.
(2) 100 µL of 1*10⁵ copies/mL influenza A virus was respectively added to each of the six virus preservation solutions in the above step (1), then divided into three groups to store the samples at 37°C, 4°C and -80°C respectively. After storage for the set days, the samples were taken to perform ultrasonic treatment for 120s at a frequency of 40 kHz. Then the samples were detected according to the specific arrangement as shown in Table 7 below.

**Table 7: Temperature settings for each group**

| Processing temperature | -80°C | | | | 4°C | | | | 37°C | | | |
|---|---|---|---|---|---|---|---|---|---|---|---|---|
| No.1 | 0d | 3d | 7d | 14d | 0d | 1d | 3d | 7d | 0d | 1d | 3d | 7d |
| No.2 | 0d | 3d | 7d | 14d | 0d | 1d | 3d | 7d | 0d | 1d | 3d | 7d |
| No.3 | 0d | 3d | 7d | 14d | 0d | 1d | 3d | 7d | 0d | 1d | 3d | 7d |
| No.4 | 0d | 3d | 7d | 14d | 0d | 1d | 3d | 7d | 0d | 1d | 3d | 7d |
| No.5 | 0d | 3d | 7d | 14d | 0d | 1d | 3d | 7d | 0d | 1d | 3d | 7d |
| No.6 | 0d | 3d | 7d | 14d | 0d | 1d | 3d | 7d | 0d | 1d | 3d | 7d |

### (4) Detection of storage effect:

According to the experimental arrangement in the above Table 7, corresponding samples were taken for detection at different time periods, and the detection was carried out according to the instructions of the universal nucleic acid detection kit for influenza A virus from Sansure Biotech Co., Ltd. The detection results at -80°C are shown in Table 8. It can be seen from Table 2 that, samples Nos. 1-3 have the best effect; after being stored at -80°C for 14 days, their detection effects are still the same as that on Day 0. Sample Nos. 4-5 have the second best effect, with the Ct values varying around 1, while sample No. 6 has the worst effect.

**Table : Comparison of Ct values of samples stored at -80°C with different preservation solutions**

| Processing temperature | Storage at -80°C | | | |
|---|---|---|---|---|
| Processing time | 0d | 3d | 7d | 14d |
| No.1 | 34.63 | 34.74 | 34.8 | 34.73 |
| No.2 | 34.55 | 34.6 | 34.9 | 34.99 |
| No.3 | 34.74 | 34.79 | 34.96 | 34.81 |
| No.4 | 35.31 | 36.17 | 36.32 | 38.15 |
| No.5 | 36.35 | 36.55 | 37.13 | 37.36 |
| No.6 | 36.85 | 37.52 | 38.01 | 39.3 |

The detection results at 4°C are shown in Table 3. It can be seen from Table 9 that, samples Nos. 1-3 have the best effects, after being stored at 4°C for 7 days, their detection effects are still the same as on Day 0. The effects of samples Nos. 4-5 are second best, and after 7 days of storage, the Ct values are delayed by more than 3, while sample No. 6 has the worst effect and could no longer be detected after 7 days of storage, indicating that its storage effect is not good.

**Table 9: Comparison of Ct values of samples stored at low temperature (4°C) using different formulas of preservation solutions.**

| Processing temperature | Storage at 4°C | | | |
|---|---|---|---|---|
| Processing time | 0d | 1d | 3d | 7d |
| No.1 | 34.72 | 34.89 | 35.05 | 35.01 |
| No.2 | 34.67 | 34.94 | 35.18 | 35.45 |
| No.3 | 35.00 | 35.10 | 35.34 | 35.55 |
| No.4 | 35.92 | 37.04 | 37.93 | 39.05 |
| No.5 | 35.47 | 37.01 | 37.04 | 39.05 |
| No.6 | 36.05 | 37.61 | 38.79 | NoCt |

The detection results at 37°C are shown in Table 4. It can be seen from Table 10 that, samples Nos. 1-3 have the best effect, after being stored at 37°C for 7 days, their detection effects are still the same as that on Day 0. The effects of samples Nos. 4-5 are not good, and after 3 days of storage, the Ct values are delayed by more than 2, while sample No. 6 has the worst effect and could no longer be detected after 3 days of storage, indicating that its storage effect is not good.

**Table 10: Comparison of Ct values of samples stored at 37°C using different formulas of preservation solutions**

| Processing temperature | Storage at 37°C | | | |
|---|---|---|---|---|
| Processing time | 0d | 1d | 3d | 7d |
| No.1 | 34.97 | 35.27 | 35.45 | 35.90 |
| No.2 | 34.51 | 34.67 | 34.72 | 35.07 |
| No.3 | 34.30 | 34.65 | 34.81 | 35.00 |
| No.4 | 35.69 | 36.85 | 37.70 | 39.06 |
| No.5 | 36.18 | 37.83 | 38.51 | NoCt |
| No.6 | 36.69 | 39.09 | NoCt | NoCt |

## Claims

1. A nucleic acid detection method, **characterized by** comprising the following steps:
S1, mixing a to-be-tested sample with a direct amplification preservation solution, and performing ultrasonic treatment to obtain a sample nucleic acid solution;
S2, adding the sample nucleic acid solution obtained in step S1 into an amplification reaction solution to obtain a mixed solution;
S3, carrying out amplification reaction by the mixed solution, and detecting nucleic acids;
wherein the direct amplification preservation solution comprises: a protective agent, a nuclease inhibitor, a surfactant, a buffer system, and a bacteriostatic agent.

2. The nucleic acid detection method according to claim 1, **characterized in that** the protective agent comprises sucrose, choline chloride, mannitol, polyvinyl pyrrolidone, and glycerol.

3. The nucleic acid detection method according to claim 2, wherein in the direct amplification preservation solution, the concentration of sucrose is 0.15-1.5% (w/v), the concentration of choline chloride is 1-10 mM, the concentration of polyvinyl pyrrolidone is 1-10 mM, the concentration of mannitol is 0.045-0.45% (v/v), and the concentration of glycerol is 0.05-0.5% (v/v).

4. The nucleic acid detection method according to claim 1, wherein the concentration of the nuclease inhibitor is 0.01-0.1% (v/v), the concentration of the surfactant is 0.01-0.5% (v/v), the concentration of the bacteriostatic agent is 0.01-0.1% (v/v), and the concentration of the buffer system is 80-120 mM.

5. The nucleic acid detection method according to claim 1, **characterized in that** the frequency of the ultrasound is 30-80 kHz, and the time is 30-150 S.

6. The nucleic acid detection method according to claim 1, **characterized in that**, in step S1, the to-be-tested sample is mixed with the direct amplification preservation solution in a container with a pipetting function; and the sample nucleic acid solution is directly added to the amplification reaction premix through the container.

7. The nucleic acid detection method according to claim 1, **characterized in that**, in step S2, the amplification reaction solution is a PCR reaction solution, and the PCR reaction solution comprises any one or more of: primers, probes, Mg²⁺, dNTPs, Taq enzyme, reverse transcriptase, and PCR buffer.

8. The nucleic acid detection method according to claim 6, wherein the PCR reaction solution comprises Mg²⁺ 4mM-8mM, dNTPs 0.2-0.6mM, Taq enzyme 5-30U, primers 200-600nM, probes 100-500nM, and RNasin 1-20U.

9. The nucleic acid detection method according to claim 1, **characterized in that**, in step S3, the detection is PCR, and the time of the PCR does not exceed 60 min.

10. The nucleic acid detection method according to claim 9, wherein the PCR procedure is as follows:
| Procedure | | |
|---|---|---|
| Temperature | Time | Number of cycles |
| 50°C | 3min | 1 |
| 95°C | 30s | 1 |
| 95°C | 10s | 45 |
| 60°C | 20s | |

11. A sample preservation composition, **characterized by** comprising a protective agent, wherein the protective agent comprises sucrose, choline chloride, mannitol, polyvinyl pyrrolidone, and glycerol.

12. The sample preservation composition according to claim 11, **characterized in that**, in the sample preservation composition, the concentration of sucrose is 0.15-1.5% (w/v), the concentration of choline chloride is 1-10 mM, the concentration of polyvinyl pyrrolidone is 1-10 mM, the concentration of mannitol is 0.045-0.45% (v/v), and the concentration of glycerol is 0.05-0.5% (v/v).

13. The sample preservation composition according to claim 12, **characterized in that** the sample preservation composition further comprises any one or more of: a nuclease inhibitor, a surfactant, a buffer system, and a bacteriostatic agent.

14. The sample preservation composition according to claim 13, wherein the nuclease inhibitor is at least one selected from the group consisting of: RNasin, diethyl pyrophosphate, or guanidine isothiocyanate; the surfactant is at least one of: Triton X-100, Tween 20, sodium dodecyl alcohol ether sulfate, or sodium dodecyl sulfate; the buffer system is at least one of: Tris-HCl buffer, phosphate buffer, or TE buffer; and the bacteriostatic agent is at least one of: Proclin 300 or potassium sorbate.

15. The sample preservation composition according to claim 14, wherein the nuclease inhibitor is RNasin; the surfactant is Triton X-100 and Tween 20; the buffer system is Tris-HCl buffer; and the bacteriostatic agent is Proclin 300.

16. The sample preservation composition according to any one of claims 13-15, wherein the concentration of the nuclease inhibitor is 0.01-0.1% (v/v), the concentration of the surfactant is 0.01-0.5% (v/v), the concentration of the bacteriostatic agent is 0.01-0.1% (v/v), and the concentration of the buffer system is 80-120mM.

17. An extraction-free nucleic acid detection kit, **characterized by** comprising the sample preservation composition according to any one of claims 11-16.

18. The extraction-free nucleic acid detection kit according to claim 17, **characterized in that** the detection kit is a PCR detection kit.

19. Use of the sample preservation composition according to any one of claims 11-16 in the preparation of an extraction-free nucleic acid detection kit.

20. The use according to claim 19, **characterized in that** the extraction-free nucleic acid detection kit is an extraction-free PCR detection kit.
